# FASCICULE DE BREVET EUROPEEN

(11) **EP 0 461 189 B1**
(45) Date de publication et mention de la délivrance du brevet: **09.06.1993**
(21) Numéro de dépôt: 90904642.7
(22) Date de dépôt: 01.03.1990
(51) Int. Cl.: A61K 7/06

(54) **COMPOSITION COSMETIQUE CAPILLAIRE CONTENANT UNE GLYCOPROTEINE**
KOSMETISCHE ZUBEREITUNG FÜR HAARWUCHS ENTHALTEND GLYKOPROTEIN
COSMETIC COMPOSITION FOR HAIR GROWTH CONTAINING A GLYCOPROTEIN

(30) Priorité: 03.03.1989 FR 8902830
(43) Date de publication de la demande: 18.12.1991
(73) Titulaire: COLETICA, 69007 Lyon (FR)
(72) Inventeur: HUC, Alain, F-69110 Ste-Foy-lès-Lyon (FR); ANTONI, Danielle, F-69390 Vernaison (FR)
(74) Mandataire: Portal, Gérard
(86) Numéro de dépôt international: FR9000143
(87) Numéro de publication internationale: WO9009778

(56) Documents cités:
- EP-A- 0 182 756
- EP-A- 0 279 244
- EP-A- 0 279 244
- EP-A- 0 297 455
- FR-A- 2 246 272
- FR-A- 2 614 787
- Patent Abstracts of Japan, vol. 8, no. /178 (C-238)(1615), 16 August 1984
- Patent Abstracts of Japan, vol. 11, no. /85 (C-410)(2532), 14 March 1987
- Patent Abstracts of Japan, vol. 11, no. 353 7 (C-457)(2800), 18 novembre 1987
- Chemical Abstracts, vol. 105, no. 18, 3 novembre 1986, (Columbus, Ohio, US), T.D. Kim et al.: "Studies on liposome-encapsulated heparin", voir page 364, résumé 158688b, & Throm. Res. 1986, 43(6), 603-12

## Description

La présente invention concerne essentiellement une composition cosmétique capillaire contenant une glycoprotéine.

Plus particulièrement, la présente invention concerne une composition de base pour la préparation d'une composition cosmétique formant composition capillaire, une composition cosmétique formant composition capillaire ainsi préparée, un procédé de préparation de la composition de base ou de la composition cosmétique et l'utilisation d'une glycoprotéine seule ou combinée à d'autres substances actives telles que l'héparine, l'héparane sulfate ou l'acide nicotinique ou ses sels, pour la préparation d'une composition cosmétique capillaire pour le traitement du cheveu.

On connaît déjà de nombreuses compositions cosmétiques formant lotion capillaire pour le traitement du cuir chevelu. Selon certaines propositions antérieures, ces compositions cosmétiques renferment des mucopolysaccharides seuls ou associés à un extrait placentaire. Par exemple, le document FR-A-2 574 289 décrit la combinaison de mucopolysaccharides acides extraits du tissu conjonctif d'animaux, d'un protéolysat cellulaire bactérien, de peptides de sérum de veau, et de peptides embryonnaires, avec un extrait placentaire issu d'animaux.

Cependant, les mucopolysaccharides utilisés et notamment ceux commercialisés sous le nom de Trichopeptides® cités en page 4, lignes 17 à 21 ne contiennent pas, ou seulement des traces, d'héparine ou d'héparane sulfate. Il en est de même des extraits placentaires d'origine bovine décrits dans ce document (page 4, lignes 8 à 11 et 15-16).

D'autres documents qui décrivent l'incorporation de mucopolyssacharides dans des compositions pour le traitement des cheveux comprennent EP-A-035 919. Parmi les mucopolyssacharides, on cite le chondroïtine-4-sulfate (page 2, lignes 14-15).

On peut encore citer FR-A-2 588 756, FR-A-2 591 889, FR-A-1 582 828, JP-A-59-186 911, JP-A-103-150 210, EP-A-182 756, et EP-A-2 094 109. Cependant, dans aucun des documents de l'état de la technique antérieure, on n'utilise pas de l'héparine ou de l'héparane sulfate en quantité significative.

On peut expliquer l'emploi des mucopolysaccharides préconisés par les documents de la technique antérieure par le fait qu'ils améliorent la circulation sanguine dans le follicule pileux grâce à une plus grande fluidité du sang et à une influence sur la reconstitution des microvaisseaux, les glycosaminoglycannes ayant des propriétés angiogénétiques. Les mucopolysaccharides ont également une influence sur le développement et la différenciation des cellules produisant la kératine du cheveu. Egalement, les glycosamonoglycannes sulfatés ont une action comme donneur de soufre lors de la synthèse de la kératine du cheveu.

On connaît encore par le document EP-A-297 455 des compositions ayant une activité de stimulation capillaire comprenant des mélanges consistant essentiellement de sulfomucopolyssacharides ayant un domaine de compositions déterminées de glycosaminoglycannes individuels parmi lesquels on cite dans les revendications l'héparine de 2 à 30 %. en poids, ou l'héparane sulfate de 10 à 40 % en poids.

En outre, le document EP-A-279 244 TONFER décrit une composition pour le traitement et la stimulation de la croissance des cheveux comprenant comme ingrédients actifs un composé choisi parmi l'héparine et/ou la vitamine K et/ou le clofibrate, la teneur en héparine étant au maximum de 0,1 % (page 5, lignes 54-56).

La présente invention a pour but de résoudre le nouveau problème technique consistant en la fourniture d'une solution permettant d'améliorer encore l'efficacité des compositions cosmétiques constituant des compositions capillaires pour le traitement des cheveux, notamment par amélioration du développement cellulaire et de la régénération des tissus et donc de la kératine du cheveu.

La présente invention a encore pour but de résoudre le nouveau problème technique consistant en la fourniture d'une solution permettant de prolonger l'effet des principes actifs dans l'organisme en améliorant encore l'efficacité de traitement, et en améliorant l'adhésion entre les constituants de la composition et les membranes cellulaires en renforçant ainsi le développement des cellules du bulbe pileux en amenant une synthèse accrue de kératine.

La présente invention a encore pour but de résoudre le nouveau problème technique consistant en la fourniture d'une solution permettant d'améliorer la disponibilité des principes actifs, et de favoriser la microcirculation au niveau bulbe pileux.

Tous ces problèmes techniques sont résolus pour la première fois d'une manière particulièrement simple, peu coûteuse, utilisable à l'échelle industrielle.

Ainsi, selon un premier aspect, la présente invention couvre une composition de base pour la préparation d'une composition cosmétique formant composition capillaire, comprenant au moins une glycoprotéine, caractérisée en ce qu'elle comprend en outre du collagène ou atélocollagène.

Selon encore une autre variante de réalisation avantageuse de l'invention, la quantité en glycoprotéine est comprise entre 10⁻⁴ % et 10⁻¹ % en poids de la composition de base finale.

Selon une autre variante de réalisation particulière, la concentration en glycoprotéine est comprise entre 0,5 et 2% en poids par rapport au collagène ou atélocollagène.

Selon une variante de réalisation particulière de l'invention, la glycoprotéine précitée est choisie parmi la fibronectine, la laminine, ou leurs mélanges en toute proportion.

Selon un mode de réalisation particulier, la fibronectine est obtenue à partir de plasma. D'autre part, la laminine est avantageusement obtenue à partir du placenta.

Selon une autre caractéristique avantageuse de l'invention, la composition est caractérisée en ce qu'elle comprend au moins une substance active choisie parmi le groupe consistant de l'héparine ou de l'héparane sulfate, de préférence, entre 1 et 5 % en poids de la composition de base finale.

Selon un mode de réalisation particulièrement avantageux de l'invention, la composition est caractérisée en ce qu'elle comprend en outre un agent activateur de la microcirculation, en particulier un sel de l'acide nicotinique, tel que le nicotinate de méthyle, avantageusement à une concentration comprise entre environ 1 et 5 % en poids de la composition de base finale.

Selon un autre mode de réalisation particulièrement avantageux, cette composition comprend en outre un ou plusieurs mucopolysaccharides ou glycosaminoglycannes, la proportion de l'héparine ou de l'héparane sulfate relativement à l'ensemble des mucopolysaccharides ou glycosaminoglycannes étant d'au moins 20 % en poids.

Selon un autre mode de réalisation particulièrement avantageux de l'invention, l'héparine ou l'héparane sulfate est isolé du placenta après digestion enzymatiques des protéines par une protéase appropriée. Une protéase préférée est la papaïne. Le placenta est de préférence lyophilisé avant d'être soumis à la digestion enzymatique avec la protéase. Avantageusement, l'extrait brut est purifié de manière à obtenir une proportion en héparane sulfate au moins égale à 40 % en poids par rapport à la totalité de l'extrait purifié.

Selon un autre mode de réalisation particulièrement avantageux de l'invention, au moins une substance active est au moins en partie encapsulée dans des vésicules type liposome.

Selon un mode de réalisation avantageux, les vésicules type liposome, incorporant au moins en partie ladite substance active, en particulier l'héparine ou l'héparane sulfate, une combinaison de l'héparine ou de l'héparane sulfate, sous forme pure ou sous forme d'extrait placentaire brut ou purifié, en présence de la glycoprotéine, sont stabilisées par une solution stabilisante homogène, collagène ou atélocollagène-glycosaminoglycannes, notamment en étant préparées en présence de cette solution de stabilisation.

En particulier, il s'agit d'une solution homogéne de glycosaminoglycanne et d'atélocollagène. La solution de glycosaminoglycanne est préparée par mise en solution du glycosaminoglycanne dans une solution aqueuse basique dont le pH est réglé de sorte qu'après mélange avec la solution d'atélocollagène, le pH du mélange constituant le support stabilisant reste légèrement basique tout en étant proche de la neutralité. De préférence, ce pH final est voisin de 8. La solution aqueuse basique peut être avantageusement une solution aqueuses de soude.

La concentration en glycosaminoglycanne dans la solution de glycosaminoglycanne est avantageusement comprise entre 0,5 et 4 %, encore mieux entre 0,5 et 2 % et encore de préférence est voisine de 1 %.

Par ailleurs, la solution d'atélocollagène est avantageusement une solution aqueuse d'atélocollagène ayant de préférence une concentration comprise entre 0,5 et 2 % en poids, et encore de préférence est voisine de 1 %. Cette solution d'atélocollagène peut être obtenue par dissolution de fibres d'atélocollagène dans une solution aqueuse légèrement acide. Selon une variante avantageuse, les fibres d'atélocollagène sont mises en solution dans de l'acide acétique 0,1 %. Selon une variante, l'atélocollagène peut également être obtenu par digestion enzymatique de collagène. La suspension des vésicules type liposome peut être introduite dans la solution de support stabilisant atélocollagène-glycosaminoglycanne selon des volumes sensiblement égaux, la proportion en vésicule type liposome étant avantageusement comprise entre 0,5 et 2 % de la composition finale (exprimée en proportions de lipides constituant la paroi lipidique des vésicules type liposome).

Selon une autre variante, les vésicules type liposome sont préparées en présence du support stabilisant collagène-glycosaminoglycanne et en présence de la glycoprotéine précitée. Ces composés participent à la constitution de la membrane lipidique des liposomes.

Ainsi, selon encore un autre mode de réalisation particulièrement avantageux de l'invention, on peut prévoir d'ajouter la glycoprotéine dans le support stabilisant d'atélocollagène-glycosaminoglycanne, la concentration en glycoprotéine dans le support stabilisant étant de préférence comprise entre 0,5 et 2 % en poids par rapport au collagène.

Selon encore une autre variante de réalisation de l'invention, à titre de mucopolysaccharides ajouté à l'héparine ou à l'héparane sulfate, on peut utiliser l'acide hyaluronique, le chondroïtne-4-sulfate, le chondroïtine-6-sulfate, le dermatane-sulfate, le kératane sulfate ou un mucoïtine sulfate.

Selon un deuxième aspect, la présente invention couvre également une composition cosmétique formant composition capillaire, pour le traitement des cheveux, caractérisée en ce qu'elle est préparée à partir d'une composition de base telle que précédemment définie.

Selon une variante de réalisation avantageuse, la composition cosmétique est finalement préparée à partir de la composition de base en diluant la suspension de vésicules type liposome dans une solution aqueuse formant un tampon citrate. Cette dilution est, par exemple, de 10 fois. De préférence, dans ce tampon citrate, il est ajouté un alkyléneglycol, en particulier le propylèneglycol.

Le tampon citrate ainsi que l'alkylèneglycol comme le propylèneglycol, améliorent l'homogénéité de la composition capillaire. Grâce à l'invention, on améliore de manière particulièrement inattendue le développement cellulaire et la régénération des tissus, donc de la kératine du cheveu. La formulation en vésicules type liposome permet de faciliter la pénétration de l'héparane sulfate ou de l'héparine au contact de la membrane des cellules synthétisant la kératine du cheveu. En outre, grâce à des effets de synergie réalisés en combinant l'atélocollagène-glycosaminoglycanne seul ou en outre avec la glycoprotéine, on améliore la prolifération cellulaire ainsi que l'adhésion des constituants de la composition selon l'invention à la membrane cellulaire. Il a pu être observé que la glycoprotéine comporte des sites d'interaction spécifiques avec la membrane cellulaire et avec la fibre collagène. La composition selon l'invention permet ainsi de renforcer de manière inattendue le développement des cellules de bulbe pileux et d'accroître la synthèse de kératine.

Par ailleurs, un autre effet de synergie réside dans le fait que la glycoprotéine, en particulier la fibronectine ou la laminine, est stabilisée par le collagène (atélocollagène) de sorte que cette glycoprotéine est maintenue à l'état natif aussi bien dans la composition que lorsqu'elle est appliquée sur les cheveux.

Par ailleurs, l'utilisation d'un sel de l'acide nicotinique, en particulier sous une forme au moins en partie incorporée dans les vésicules type liposome, permet d'améliorer la microcirculation au niveau du bulbe pileux.

Il est à observer qu'un autre effet particulièrement inattendu de l'emploi de la combinaison héparane sulfate ou héparine, sous forme pure ou sous forme d'extrait placentaire brut ou purifiée, avec une glycoprotéine, réside dans la prolifération cellulaire résultante du piégeage des facteurs de croissance au niveau du bulbe pileux et de la combinaison collagène ou atélocollagène-mucopolysaccharide-glycoprotéine.

### Exemple 1

### 1) Préparation de l'extrait mucopolysaccharide placentaire renfermant l'héparane sulfate.

### A) Réactifs.

a) Enzyme
   L'enzyme utilisée est la papaïne "Merck" à 30 000 U.I./mg.
b) Tampon KCl
   Sa composition est la suivante :
   - Chlorure de potassium: 11,8 g/l
   - Cystéine: 78,8 mg/l
   - EDTA: 180 mg/l

   Le pH est ajusté à 6,5.

### B) Méthode.

Le placenta ovin récupéré juste après la naissance est lyophilisé.

300 g d'enzyme sont dissous dans 225 l de tampon KCl. 30 kg de placenta lyophilisé sont alors introduits dans ce bain.

La température du bain est amenée à 60°C et elle est maintenue à ce niveau durant toute l'opération de digestion enzymatique. Aprés 24 h, une quantité d'enzyme égale à celle utilisée est ajoutée au bain réactionnel.

Après 24 h supplémentaires, le bain est décanté par gravité naturelle et le surnageant est centrifugé à l'aide d'une décanteuse Robatel tournant à 3 000 tr/min et donnant une accélération de 2 000 g.

La température du liquide obtenue est abaissée à 15°C. Est alors ajoutée au bain maintenu sous agitation une quantité d'acide trichloracétique telle qu'une concentration de 3,5 % soit obtenue. L'ensemble est laissé au repos pendant une dizaine d'heures. Le précipité obtenu par gravité naturelle est éliminé et les particules solides restant dans le surnageant sont séparées par la décanteuse dans les mêmes conditions que celles décrites précédemment.

Le liquide obtenu est alors neutralisé par la soude. Le bain est laissé au repos pendant 3 h.

Après décantation et centrifugation, le surnageant est ultrafiltré à l'aide d'un appareil d'ultrafiltration Millipore comportant des membranes sont le seuil de coupure est de 10 000 daltons. L'ultrafiltrat est lyophilisé. Environ 500 g d'extrait mucopolysaccharide placentaire sont obtenus.

### 2) Purification de l'héparane sulfate

Dans 15 l d'eau permutée, sont ajoutés 1 kg de résine Dowex® (1 x 2, forme Cl-, 100-200 mesh) et 1,5 kg d'extrait placentaire obtenu précédemment. Après une agitation de 1 h, la résine est décantée par gravité naturelle et le surnageant est rejeté. La résine est alors placée dans un bain contenant du NaCl 0,75 M.
1er bain
   Après une agitation de 1 h, la résine est décantée de façon naturelle et le surnageant est envoyé dans l'ultrafiltreur.
   L'ultrafiltrat obtenu est lyophilisé. De cette manière, environ 150 g d'acide hyaluronique sec sont préparés.
2e bain
   La résine est alors placée dans un bain contenant du NaCl 1,6 M. Après même processus que précédemment, 450 g d'héparane sulfate sec sont obtenus.
3e bain
   La résine est enfin mise en contact avec un bain renfermant du NaCl 3 M. Après le même processus que précédemment, 30 g de dermatane sulfate contaminés par un peu d'héparane sont obtenus sous forme sèche.

### 3) Préparation du collagène déréticulé

La peau de veau fraîchement abattu est soumise à épilation chimique dans un bain contenant 3 % de sulfure de sodium et 4 % de chaux, la proportion étant de 100 g de peau pour 200 cm³ de solution. Le derme est alors isolé du reste de la peau par une opération de refendage grâce à une scie tournante à ruban.

Le tissu obtenu est broyé et extrudé à travers une grille comportant des trous de 4 mm. Le broyat est alors mis en contact pendant 3 semaines avec un lait de chaux saturé à raison de 1 kg pour 4 l de solution. La peau ainsi traitée est séparée du surnageant par une centrifugation en continu sous une accélération de 2 000 g à l'aide d'une décanteuse tournant à 4 000 tr/min. Le culot est ensuite soumis à deux lavages à l'eau courante dans une cuve inox sous agitation lente à raison de 1 kg pour 4 l de bain. Le broyat est alors soumis à deux traitements du tampon phosphate pH 7,8 (21,7 g/l de Na₂HPO₄ et 0,78 g/l de KH₂PO₄) dans les mêmes conditions que pour le lavage à l'eau. Le culot est alors lavé par deux bains d'eau désionisée et stérile. Le broyat obtenu est placé dans une solution d'acide acétique (0,5 g/l, pH 3,4) à raison de 1 kg pour 20 l de bain. Après 5 min d'agitation, le surnageant est séparé du culot par décantation en continu selon la technique précédente. Le collagène est alors précipité par le surnageant par addition de chlorure de sodium sec dans une proportion de 10 % environ par rapport au bain. Après décantation par gravité, les fibres obtenues sont dialysées contre de l'eau désionisée et stérile à l'aide de membranes de dialyse, de préférence formées par des boyaux dont le seuil de coupure est compris entre 6 000 et 8 000 daltons. Après avoir vérifié par le nitrate d'argent que les fibres dialysées ne contenaient plus de chlorure de sodium, celles-ci sont mises en solution dans un bain renfermant 6 g/l d'acide acétique de façon à obtenir une concentration finale de 1 % en protéine. L'ensemble est agité sous agitation lente pendant 24 h. 1 kg de broyat conduit à 20 l de solution environ.

### 4) Préparation du chondroïtine-4-sulfate

Des cloisons nasales de veau débarrassées des tissus musculaires et adipeux sont hachées et broyées par extrusion à travers une grille comportant des trous de 4 mm ; le broyat est placé pendant 24 h à une température de 6°C dans un tampon de chlorure de potassium (11,8 g/l de KCl, 78,8 mg/l de cystéine, ETDA 180 mg/l) renfermant 1 % de papaïne "Merck". La proportion étant de 130 g de broyat pour 1 l de tampon.

Le surnageant est séparé du culot par centrifugation en continu à l'aide d'une décanteuse tournant à 4 000 tr/min. Au surnageant sont alors ajoutés 40 g/l d'acide trichloracétique. Le précipité est éliminé par centrifugation en continu selon la technique précédente. Le surnageant est neutralisé à l'aide de soude en pastille. Le mélange est alors dialysé contre de l'eau désionisée et stérile à l'aide de boyaux dont le seuil de coupure est compris entre 6 000 et 8 000 daltons. La solution dialysée est lyophilisée. Le chondroïtine-4-sulfate est obtenu à l'état sec. 1 kg de cloisons nasales permet d'obtenir 90 g de mucopolysaccharides.

### 5) Préparation d'une composition capillaire

### A) Solution liposomique de base

Dans 10 kg de complexe collagène glycosaminoglycanne renfermant 20 g de collagène, 5 g de chondroïtine-4-sulfate ainsi que des conservateurs, par exemple 10 g de Nipagin® et 50 g de Phénonip® de chez NIPA Lab. Ltd/UK, on ajoute par exemple 10 g d'héparane sulfate et 10 g de nicotinate de méthyle. L'ensemble est agité sous agitation mécanique jusqu'à obtention d'une solution homogène, le pH est ajusté à 7,2.

Dans ce bain maintenu sous agitation à température ambiante, sont ajoutés 100 g d'un mélange homogène de lécithine et de cholestérol. Après dissolution complète, le mélange est agité par agitateur ultrason type ultra Turax type UTL T60 à 8 000 tr/min pendant 10 min, sont alors ajoutés 0,2 % de nicotinate de méthyle et 0,002 % de fibronectine native extraite du plasma bovin. L'ensemble est homogénéisé sous agitation douce.

### B) Composition capillaire finale

10 kg de la solution liposomique précédente sont dilués dans 50 l d'eau permutée stérile. Dans cette solution sont alors ajoutés 5 kg de propylèneglycol, 100 g de Nipagin® et 500 g de Phénonip® ainsi que 200 g de triacétate de sodium. Le volume de bain est alors complété à 100 l et le pH est ajusté à 7,8 à l'aide d'acide citrique.

La composition ainsi obtenue est légèrement trouble opalescente et de couleur blanche faiblement jaune.

### Exemple 2

La méthodologie est exactement la même que précédemment, mais l'héparane pur est remplacé par l'extrait mucopolysaccharidique placentaire. Mais dans ce cas, la quantité de solution liposomique sera doublée. C'est-à-dire que sa concentration dans la composition finale sera de 20 % au lieu de 10 % précédemment.

### Exemple 3

La composition capillaire est identique à celle de l'exemple 1, sauf en ce qui concerne l'héparane sulfate qui est remplacé par l'héparine.

### Exemple 4

### ESSAIS DE REPOUSSE DES POILS

La repousse du poil a été étudiée sur des rats traités par la composition préparée selon l'exemple 1 en comparaison avec deux animaux traités par le placébo, à savoir le mélange de tampon citrate, de propylèneglycol et de conservateur.

Pour cela, 20 cm² de la peau du dos des rats préalablement rasés ont été traités quotidiennement par 0,5 g soit de composition selon l'invention, soit de placébo. Au bout de 21 jours, il a été constaté que la repousse des poils était en moyenne deux fois plus rapide chez les rats traités par la lotion. Cette expérience a été réalisée sur deux lots de 10 rats.

Le produit peut être conditionné en ampoule de 5 ml. Cette quantité correspond à un traitement quotidien.

## Revendications (Revendications pour l'(les) Etat(s) contractant(s) suivant(s): AT, BE, CH, DE, DK, FR, GB, IT, LI, LU, NL, SE)

1. Composition de base pour la préparation d'une composition cosmétique formant composition capillaire, comprenant au moins une glycoprotéine, caractérisée en ce qu'elle comprend en outre du collagène ou atélocollagène.

2. Composition selon la revendication 1, caractérisée en ce que la quantité en glycoprotéine est comprise entre 10⁻⁴% et 10⁻¹% en poids de la composition de base finale.

3. Composition selon la revendication 1 ou 2, caractérisée en ce que la concentration en glycoprotéine est comprise entre 0,5 et 2% en poids du collagène ou atélocollagène.

4. Composition selon l'une des revendications 1 à 3, cactérisée en ce que la glycoprotéine précitée est choisie parmi la fibronectine, la laminine, ou leurs mélanges en toutes proportions.

5. Composition selon la revendication 4, caractérisée en ce que la fibronectine est obtenue à partir de plasma.

6. Composition selon la revendication 4, caractérisée en ce que la laminine est obtenue à partir de placenta.

7. Composition selon l'une des revendications 1 à 6, caractérisée en ce qu'au moins une substance active est au moins en partie encapsulée dans des vésicules type liposome.

8. Composition selon la revendication 7, caractérisée en ce que les vésicules type liposome, incorporant au moins en partie au moins une substance active, sont stabilisées par une solution de stabilisation homogéne de collagène ou d'atélocollagène-glycosaminoglycanne, qui contient au moins en partie la glycoprotéine précitée, notamment en étant préparée en présence de cette solution de stabilisation.

9. Composition selon l'une des revendications 1 à 8, caractérisé en ce qu'elle comprend au moins une substance active choisie parmi le groupe consistant de l'héparine ou de l'héparane sulfate, de préférence, la quantité en héparine ou en héparane sulfate est comprise entre 1 et 5 % en poids de la composition de base finale.

10. Composition selon l'une des revendications 1 à 9, caractérisée en ce qu'elle comprend en outre un agent activateur de la microcirculation, en particulier un sel de l'acide nicotinique, tel que le nicotinate de méthyle, avantageusement à une concentration comprise entre 1 et 5 % en poids de la composition de base finale.

11. Composition selon la revendication 9 ou 10, caractérisé en ce qu'elle comprend en outre un ou plusieurs mucopoplysaccharides ou glycosaminoglycannes, la proportion de l'héparine ou de l'héparane sulfate relativement à l'ensemble des mucopolysaccharides ou glycosamincglycannes étant d'au moins 20 % en poids.

12. Composition selon l'une quelconque des revendications 9 à 11, caractérisée en ce que l'héparine ou l'héparane sulfate est isolé du placenta après digestion enzymatique des protéines par une protéase appropriée, de préférence la papaïne, sous forme pure ou sous forme d'extrait placentaire pur ou purifié.

13. Composition selon la revendication 12, caractérisée en ce que le placenta est lyophilisé avant d'être soumis à la digestion enzymatique avec la protéase ; de préférence l'extrait brut est purifié de manière à obtenir une proportion en héparane sulfate au moins égale à 40 % en poids par rapport à la totalité de l'extrait purifié.

14. Composition selon l'une des revendications 8 à 13, caractérisée en ce que la suspension des vésicules type liposome est associée à la solution de support stabilisant atélocollagène-glycosaminoglycanne selon des volumes sensiblement égaux, la proportion en vésicules type liposome est avantageusement comprise entre 0,5 et 2 % de la composition de base finale.

15. Composition selon l'une des revendications 11 à 14, caractérisée en ce qu'on utilise comme mucopolysaccharides l'acide hyaluronique, le chondroïtine-4-sulfate, le chondroïtine-6-sulfate, le dermatane-sulfate, le kératane-sulfate ou un mucoïtine-sulfate.

16. Composition cosmétique formant composition capillaire pour le traitement des cheveux, caractérisée en ce qu'elle est préparée à partir d'une composition de base telle que définie dans l'une quelconque des revendications précédentes.

17. Composition cosmétique selon la revendication 16, caractérisée en ce qu'elle est finalement préparée à partir de la composition de base en diluant la suspension de vésicules type liposome dans une solution aqueuse formant un tampcn citrate, dans lequel il est de préférence ajouté un alkylèneglycol, en particulier le propylèneglycol, en particulier en diluant environ 10 fois.

18. Procédé de préparation d'une composition de base ou d'une composition cosmétique, caractérisé en ce qu'il comprend l'incorporation d'au moins une substance active telle que définie dans l'une quelconque des revendications 1 à 17, dans un véhicule, support ou excipient approprié, en particulier dans un véhicule, support ou excipient cosmétiquement acceptable.

19. Utilisation d'une glycoprotéine, en particulier la fibronectine ou la laminine, en combinaison avec du collagène ou atécollagène, pour la préparation d'une composition de base ou d'une composition cosmétique capillaire pour le traitement des cheveux.

## Revendications (Revendications pour l'(les) Etat(s) contractant(s) suivant(s): ES)

1. Procédé de préparation d'une composition de base pour la préparation d'une composition cosmétique formant composition capillaire, comprenant l'incorporation d'au moins une glycoprotéine dans un véhicule, support ou excipient approprié, en particulier dans un véhicule, support ou excipient cosmétiquement acceptable, caractérisé en ce qu'on incorpore en outre du collagène ou atélocollagène.

2. Procédé selon la revendication 1, caractérisé en ce que la quantité en glycoprotéine est comprise entre 10⁻⁴% et 10⁻¹% en poids de la composition de base finale.

3. Procédé selon la revendication 1 ou 2, caractérisé en ce que la concentration en glycoprotéine est comprise entre 0,5 et 2% en poids du collagène ou atélocollagène.

4. Procédé selon l'une des revendications 1 à 3, caractérisé en ce que la glycoprotéine précitée est choisie parmi la fibronectine, la laminine, ou leurs mélanges en toutes proportions.

5. Procédé selon la revendication 4, caractérisé en ce que la fibronectine est obtenue à partir de plasma.

6. Procédé selon la revendication 4, caractérisé en ce que la laminine est obtenue à partir de placenta.

7. Procédé selon l'une des revendications 1 à 6, caractérisé en ce qu'au moins une substance active est au moins en partie encapsulée dans des vésicules type liposome.

8. Procédé selon la revendication 7, caractérisé en ce que les vésicules type liposome, incorporant au moins en partie au moins une substance active, sont stabilisées par une solution de stabilisation homogène de collagène ou d'atélocollagène-glycosaminoglycanne, qui contient au moins en partie la glycoprotéine précitée, notamment en étant préparée en présence de cette solution de stabilisation.

9. Procédé selon l'une des revendications 1 à 8, caractérisé en ce qu'il comprend au moins une substance active choisie parmi le groupe consistant de l'héparine ou de l'héparane sulfate, de préférence, la quantité en héparine ou en héparane sulfate est comprise entre 1 et 5% en poids de la composition de base finale.

10. Procédé selon l'une des revendications 1 à 9, caractérisé en ce qu'il comprend en outre un agent activateur de la microcirculation, en particulier un sel de l'acide nicotinique, tel que le nicotinate de méthyle, avantageusement à une concentration comprise entre 1 et 5% en poids de la composition de base finale.

11. Procédé selon la revendication 9 ou 10, caractérisé en ce qu'il comprend en outre un ou plusieurs mucopolysaccharides ou glycosaminoglycannes, la proportion de l'héparine ou de l'héparane sulfate relativement à l'ensemble des mucopolysaccharides ou glycosaminoglycannes étant d'au moins 20% en poids.

12. Procédé selon l'une des revendications 9 à 11, caractérisé en ce que l'héparine ou l'héparane sulfate est isolé du placenta après digestion enzymatique des protéines par une protéase appropriée, de préférence la papaïne, sous forme pure ou sous forme d'extrait placentaire pur ou purifié.

13. Procédé selon la revendication 12, caractérisé en ce que le placenta est lyophilisé avant d'être soumis à la digestion enzymatique avec la protéase ; de préférence l'extrait brut est purifié de manière à obtenir une proportion en héparane sulfate au moins égale à 40% en poids par rapport à la totalité de l'extrait purifié.

14. Procédé selon l'une des rendications 8 à 13, caractérisé en ce que la suspension des vésicules type liposome est associée à la solution de support stabilisant atélocollagène-glycosaminoglycanne selon des volumes sensiblement égaux, la proportion en vésicules type liposome est avantageusement comprise entre 0,5 et 2% de la composition de base finale.

15. Procédé selon l'une des revendications 11 à 14, caractérisé en ce qu'on utilise comme mucopolysaccharides l'acide hyaluronique, le chondroïtine-4-sulfate, le chondroïtine-6-sulfate, le dermatane-sulfate, le kératare-sulfate ou un mucoïtine-sulfate.

16. Procédé de préparation d'une composition cosmétique formant composition capillaire pour le traitement des cheveux, caractérisé en ce qu'elle est préparée à partir d'une composition de base telle que définie dans l'une quelconque des revendications précédentes.

17. Procédé selon la revendication 16, caractérisé en ce qu'elle est finalement préparée à partir de la composition de base en diluant la suspension de vésicules type liposome dans une solution aqueuse formant un tampon citrate, dans lequel il est de préférence ajouté un alkylèneglycol, en particulier le propylèneglycol, en particulier en diluant environ 10 fois.

18. Utilisation d'une glycoprotéine, en particulier la fibronectine ou la laminine, en combinaison avec du collagène ou atécollagène, pour la préparation d'une composition de base ou d'une composition cosmétique capillaire pour le traitement des cheveux.

## Claims (Claims for the following Contracting State(s): AT, BE, CH, DE, DK, FR, GB, IT, LI, LU, NL, SE)

1. Basic composition for the preparation of a cosmetic composition constituting a composition for the hair, comprising at least a glycoprotein, characterized in that it further comprises collagen or atelocollagen.

2. Composition according to claim 1, characterized in that the glycoprotein content is comprised between about 10⁻⁴% and 10⁻¹% by weight of the final basic composition.

3. Composition according to claim 1 or 2, characterized in that the glycoprotein concentration is comprised between 0.5 and 2% by weight of the collagen or atelocollagen.

4. Composition according to one of claims 1 to 3, characterized in that said glycoprotein is selected from fibronectin, laminin, or mixtures thereof in all proportions.

5. Composition according to claim 4, characterized in that the fibronectin is obtained from plasma.

6. Composition according to claim 4, characterized in that the laminin is obtained from placenta.

7. Composition according to one of claims 1 to 6, characterized in that at least one active ingredient is at least partly encapsulated in liposome type vesicles.

8. Composition according to claim 7, characterized in that the liposome type vesicles, incorporating at least partly one active ingredient, are stabilized by a homogeneous stabilizing solution of collagen or atelocollagen-glycosaminoglycan, containing at least partly said glycoprotein, notably by being prepared in the presence of said stabilizing solution.

9. Composition according to claims 1 to 8, characterized in that it comprises at least one active ingredient selected from the group composed of heparin or heparan sulfate, preferably, the heparin or heparan sulfate content is comprised between about 1 and 5% by weight of the final basic composition.

10. Composition according to one of claims 1 to 9, characterized in that it further comprises a microcirculation activating agent, notably a nicotinic acid salt, such as methyl nicotinate, advantageously in a concentration comprised between 1 and 5% by weight of the final basic composition.

11. Composition according to claim 9 or 10, characterized in that it further comprises one or more mucopolysaccharides or glycosaminoglycans, the heparin or heparan sulfate content relatively to the total content of polysaccharides or glycosaminoglycans being at least 20% by weight.

12. Composition according to one of claims 9 to 11, characterized in that the heparin or heparan sulfate is isolated from the placenta after enzymatic digestion of the proteins by a suitable protease, preferably papain, in pure form or in the form of pure purified placental extract.

13. Composition according to claim 12, characterized in that the placenta is lyophilized before undergoing the enzymatic digestion with the protease; preferably the crude extract is purified so as to obtain a heparan sulfate proportion at least equal to 40% by weight with respect to the total quantity of purified extract.

14. Composition according to one of claims 8 to 13, characterized in that the suspension of lipsome type vesicles is associated to the stabilizing base solution of atelocollagen-glycosaminoglycan in substantially equal volumes, the proportion of liposome type vesicles being preferably comprised between 0.5 and 2% by weight of the final basic composition.

15. Composition according to one of claims 11 to 14, characterized in that hyaluronic acid, 4-chondroitin sulfate, 6-chondroitin sulfate, dermatan sulfate, keratan sulfate or a mucoitin sulfate is used as mucopolysaccharides.

16. Cosmetic composition constituting a composition for the treatment of the hair, characterized in that it is prepared from a basic composition such as defined in any one of the preceding claims.

17. Cosmetic composition according to claim 16, characterized in that it is finally prepared from the basic composition by diluting the suspension of lipsome type vesicles in an aqeuous solution forming a citrate buffer, in which is preferably added an alkyleneglycol, in particular the propyleneglycol, particularly diluted about 10 times.

18. Process for the preparation of a basic composition or of a cosmetic composition, characterized in that it comprises incorporating at least one active ingredient such as defined in any one of claims 1 to 17, in a suitable vehicle, base or excipient, particularly in a suitable cosmetically acceptable vehicle, base or excipient.

19. Use of a glycoprotein, notably fibronectin or laminin, in combination with collagen or atelocollagen, for the preparation of a basic cosmetic composition or of a cosmetic composition for the treatment of the hair.

## Claims (Claims for the following Contracting State(s): ES)

1. Process for the preparation of a basic composition for the preparation of a cosmetic composition constituting a composition for the hair, comprising at least a glycoprotein in a suitable vehicle, support or excipient, particularly in a cosmetically acceptable vehicle, support or excipient, characterized in that it further comprises collagen or atelocollagen.

2. Process according to claim 1, characterized in that the glycoprotein content is comprised between about 10⁻⁴% and 10⁻¹% by weight of the final basic composition.

3. Process according to claim 1 or 2, characterized in that the glycoprotein concentration is comprised between 0.5 and 2% by weight of the collagen or atelocollagen.

4. Process according to one of claims 1 to 3, characterized in that said glycoprotein is selected from fibronectin, laminin, or mixtures thereof in all proportions.

5. Process according to claim 4, characterized in that the fibronectin is obtained from plasma.

6. Process according to claim 4, characterized in that the laminin is obtained from placenta.

7. Process according to one of claims 1 to 6, characterized in that at least one active ingredient is at least partly encapsulated in liposome type vesicles.

8. Process according to claim 7, characterized in that the liposome type vesicles, incorporating at least partly one active ingredient, are stabilized by a homogeneous stabilizing solution of collagen or atelocollagen-glycosaminoglycan, containing at least partly said glycoprotein, notably by being prepared in the presence of said stabilizing solution.

9. Process according to claims 1 to 8, characterized in that it comprises at least one active ingredient selected from the group composed of heparin or heparan sulfate, preferably, the heparin or heparan sulfate content is comprised between about 1 and 5% by weight of the final basic composition.

10. Process according to one of claims 1 to 9, characterized in that it further comprises a microcirculation activating agent, notably a nicotinic acid salt, such as methyl nicotinate, advantageously in a concentration comprised between about 1 to 5% by weight of the final basic composition.

11. Process according to claim 9 or 10, characterized in that it further comprises one or more mucopolysaccharides or glycosaminoglycans, the heparin or heparan sulfate content relatively to the total content of polysaccharides or glycosaminoglycans being at least 20% by weight.

12. Process according to one of claims 9 to 11, characterized in that the heparin or heparan sulfate is isolated from the placenta after enzymatic digestion of the proteins by a suitable protease, preferably papain, in pure form or in the form of pure purified placental extract.

13. Process according to claim 12, characterized in that the placenta is lyophilized before undergoing the enzymatic digestion with the protease; preferably the crude extract is purified so as to obtain a heparan sulfate proportion at least equal to 40% by weight with respect to the total quantity of purified extract.

14. Process according to one of claims 8 to 13, characterized in that the suspension of lipsome type vesicles is associated to the stabilizing base solution of atelocollagen-glycosaminoglycan in substantially equal volumes, the proportion of liposome type vesicles being preferably comprised between 0.5 and 2% by weight of the final basic composition.

15. Process according to one of claims 11 to 14, characterized in that hyaluronic acid, 4-chondroitin sulfate, 6-chondroitin sulfate, dermatan sulfate, keratan sulfate or a mucoitin sulfate is used as mucopolysaccharides.

16. Process for the preparation of a cosmetic composition constituting a composition for the treatment of the hair, characterized in that it is prepared from a basic composition such as defined in any one of the preceding claims.

17. Process according to claim 16, characterized in that it is finally prepared from the basic composition by diluting the suspension of lipsome type vesicles in an aqeuous solution forming a citrate buffer, in which is preferably added an alkyleneglycol, in particular the propyleneglycol, particularly diluted about 10 times.

18. Use of a glycoprotein, notably fibronectin or laminin, in combination with collagen or atelocollagen, for the preparation of a basic composition or of a cosmetic composition for the treatment of the hair.

## Patentansprüche (Patentansprüche für folgende(n) Vertragsstaat(en): AT, BE, CH, DE, DK, FR, GB, IT, LI, LU, NL, SE)

1. Basiszusammensetzung zur Herstellung einer kosmetischen Zusammensetzung in Form eines Haarmittels, die mindestens ein Glykoprotein enthält, dadurch gekennzeichnet, daß sie außerdem Collagen oder Atelocollagen enthält.

2. Zusammensetzung nach Anspruch 1, dadurch gekennzeichnet, daß die Menge an Glykoprotein zwischen 10⁻⁴ und 10⁻¹ Gew.% der fertigen Basiszusammensetzung beträgt.

3. Zusammensetzung nach den Ansprüchen 1 oder 2, dadurch gekennzeichnet, daß die Konzentration an Glykoprotein zwischen 0,5 und 2 Gew.% des Collagens oder Atelocollagens beträgt.

4. Zusammensetzung nach einem der Ansprüche 1 bis 3, dadurch gekennzeichnet, daß das genannte Glykoprotein ausgewählt ist aus Fibronektin, Laminin oder Mischungen davon in sämtlichen Verhältnissen.

5. Zusammensetzung nach Anspruch 4, dadurch gekennzeichnet, daß das Fibronektin aus Plasma erhalten ist.

6. Zusammensetzung nach Anspruch 4, dadurch gekennzeichnet, daß das Laminin aus der Plazenta erhalten ist.

7. Zusammensetzung nach einem der Ansprüche 1 bis 6, dadurch gekennzeichnet, daß mindestens eine aktive Substanz zumindest zum Teil in Bläschen vom Liposomentyp eingekapselt ist.

8. Zusammensetzung nach Anspruch 7, dadurch gekennzeichnet, daß die Bläschen vom Liposomentyp, die zumindest zum Teil mindestens eine aktive Substanz enthalten, mittels einer homogenen Stabilisationslösung aus Collagen- oder Atelocollagen-glycosaminoglycan, die zumindest zum Teil das genannte Glykoprotein enthält, stabilisiert werden, insbesondere indem sie in Gegenwart dieser Stabilisationslösung hergestellt werden.

9. Zusammensetzung nach einem der Ansprüche 1 bis 8, dadurch gekennzeichnet, daß sie mindestens eine aktive Substanz, ausgewählt aus der Gruppe bestehend aus Heparin oder Heparansulfat, enthält und die Menge an Heparin oder an Heparansulfat vorzugsweise zwischen 1 und 5 Gew.% der fertigen Basiszusammensetzung beträgt.

10. Zusammensetzung nach einem der Ansprüche 1 bis 9, dadurch gekennzeichnet, daß sie weiters einen Aktivator für die Mikrozirkulation, insbesondere ein Salz der Nikotinsäure, wie Methylnikotinat, vorteilhafterweise in einer Konzentration zwischen 1 und 5 Gew.% der fertigen Basiszusammensetzung, enthält.

11. Zusammensetzung nach Anspruch 9 oder 10, dadurch gekennzeichnet, daß sie weiters ein oder mehrere Mukopolysaccharide oder Glykosaminoglykane enthält, wobei der Anteil an Heparin oder Heparansulfat in bezug auf die Gesamtheit der Mukopolysaccharide oder Glykosaminoglykane mindestens 20 Gew.% beträgt.

12. Zusammensetzung nach einem der Ansprüche 9 bis 11, dadurch gekennzeichnet, daß das Heparin oder Heparansulfat nach dem enzymatischen Aufschluß der Proteine mittels einer geeigneten Protease, vorzugsweise Papain, in reiner Form oder in Form eines reinen oder gereinigten Plazentaextraktes aus der Plazenta isoliert wird.

13. Zusammensetzung nach Anspruch 12, dadurch gekennzeichnet, daß die Plazenta lyophilisiert wird, bevor sie dem enzymatischen Aufschluß mit Protease unterzogen wird; vorzugsweise wird der Rohextrakt derart gereinigt, daß ein Anteil an Heparansulfat von mindestens 40 Gew.%, bezogen auf die Gesamtmenge des gereinigten Extrakts, erhalten wird.

14. Zusammensetzung nach einem der Ansprüche 8 bis 13, dadurch gekennzeichnet, daß die Suspension der Bläschen vom Liposomentyp mit der stabilisierenden Atelocollagen-glycosaminoglycan-Trägerlösung zu im wesentlichen gleichen Volumsteilen vereinigt wird, wobei der Anteil an Bläschen vom Liposomentyp vorteilhafterweise zwischen 0,5 und 2 % der fertigen Basiszusammensetzung beträgt.

15. Zusammensetzung nach einem der Ansprüche 11 bis 14, dadurch gekennzeichnet, daß man als Mukopolysaccharide Hyaluronsäure, 4-Chondroitinsulfat, 6-Chondroitinsulfat, Dermatansulfat, Keratansulfat oder Mukoitinsulfat verwendet.

16. Kosmetische Zusammensetzung in Form eines Haarmittels zur Behandlung der Haare, dadurch gekennzeichnet, daß sie hergestellt wird aus einer Basiszusammensetzung wie in einem der vorhergehenden Ansprüche definiert.

17. Kosmetische Zusammensetzung nach Anspruch 16, dadurch gekennzeichnet, daß die fertiggestellt wird aus der Basiszusammensetzung, indem die Suspension aus Bläschen vom Liposomentyp in einer einen Zitratpuffer bildenden wässerigen Lösung verdünnt wird, in den vorzugsweise ein Alkylenglykol, insbesondere Propylenglykol, zugegeben wird, insbesondere auf eine etwa 10-fache Verdünnung.

18. Verfahren zur Herstellung einer Basiszusammensetzung oder einer kosmetischen Zusammensetzung, dadurch gekennzeichnet, daß es die Einarbeitung mindestens einer aktiven Substanz, wie sie in einem der Ansprüche 1 bis 17 definiert ist, in einen geeigneten Vehikel, Träger oder Exzipienten, insbesondere einen Kosmetisch akzeptablen Vehikel, Träger oder Exzipienten, umfaßt.

19. Verwendung eines Glykoproteins, insbesondere von Fibronektin oder Laminin, in Kombination mit Collagen oder Atelocollagen zur Herstellung einer Basiszusammensetzung oder einer kosmetischen Zusammensetzung zur Behandlung der Haare.

## Patentansprüche (Patentansprüche für folgende(n) Vertragsstaat(en): ES)

1. Verfahren zur Herstellung einer Basiszusammensetzung für die Herstellung einer ein Haarmittel bildenden kosmetischen Zusammensetzung, enthaltend das Einarbeiten mindestens eines Glykoproteins in einen geeigneten Vehikel, Träger oder Exzipienten, insbesondere in einen kosmetisch akzeptablen Vehikel, Träger oder Exzipienten, dadurch gekennzeichnet, daß außerdem Collagen oder Atelocollagen eingearbeitet wird.

2. Verfahren nach Anspruch 1, dadurch gekennzeichnet, daß die Menge an Glykoprotein zwischen 10⁻⁴ und 10⁻¹ Gew.% der fertigen Basiszusammensetzung beträgt.

3. Verfahren nach den Ansprüchen 1 oder 2, dadurch gekennzeichnet, daß die Konzentration an Glykoprotein zwischen 0,5 und 2 Gew.% des Collagens oder Atelocollagens beträgt.

4. Verfahren nach einem der Ansprüche 1 bis 3, dadurch gekennzeichnet, daß das genannte Glykoprotein ausgewählt ist aus Fibronektin, Laminin oder Mischungen davon in sämtlichen Verhältnissen.

5. Verfahren nach Anspruch 4, dadurch gekennzeichnet, daß das Fibronektin aus Plasma erhalten wird.

6. Verfahren nach Anspruch 4, dadurch gekennzeichnet, daß das Laminin aus der Plazenta erhalten wird.

7. Verfahren nach einem der Ansprüche 1 bis 6, dadurch gekennzeichnet, daß mindestens eine aktive Substanz zumindest zum Teil in Bläschen vom Liposomentyp eingekapselt wird.

8. Verfahren nach Anspruch 7, dadurch gekennzeichnet, daß die Bläschen vom Liposomentyp, die zumindest zum Teil mindestens eine aktive Substanz enthalten, mittels einer homogenen Stabilisationslösung aus Collagen- oder Atelocollagen-glycosaminoglycan, die zumindest zum Teil das genannte Glykoprotein enthält, stabilisiert werden, insbesondere indem sie in Gegenwart dieser Stabilisationslösung hergestellt werden.

9. Verfahren nach einem der Ansprüche 1 bis 8, dadurch gekennzeichnet, daß es mindestens eine aktive Substanz, ausgewählt aus der Gruppe bestehend aus Heparin oder Heparansulfat, enthält und die Menge an Heparin oder an Heparansulfat vorzugsweise zwischen 1 und 5 Gew.% der fertigen Basiszusammensetzung beträgt.

10. Verfahren nach Anspruch 9, dadurch gekennzeichnet, daß es weiters einen Aktivator für die Mikrozirkulation, insbesondere ein Salz der Nikotinsäure, wie Methylnikotinat, vorteilhafterweise in einer Konzentration zwischen 1 und 5 Gew.% der fertigen Basiszusammensetzung, enthält.

11. Verfahren nach Anspruch 9 oder 10, dadurch gekennzeichnet, daß es weiters ein oder mehrere Mukopolysaccharide oder Glykosaminoglykane enthält, wobei der Anteil an Heparin oder Heparansulfat in bezug auf die Gesamtheit der Mukopolysaccharide oder Glykosaminoglykane mindestens 20 Gew.% beträgt.

12. Verfahren nach einem der Ansprüche 9 bis 11, dadurch gekennzeichnet, daß das Heparin oder Heparansulfat nach dem enzymatischen Aufschluß der Proteine mittels einer geeigneten Protease, vorzugsweise Papain, in reiner Form oder in Form eines reinen oder gereinigten Plazentaextraktes aus der Plazenta isoliert wird.

13. Verfahren nach Anspruch 12, dadurch gekennzeichnet, daß die Plazenta lyophilisiert wird, bevor sie dem enzymatischen Aufschluß mit Protease unterzogen wird; vorzugsweise wird der Rohextrakt gereinigt, daß ein Anteil an Heparansulfat von mindestens 40 Gew.%, bezogen auf die Gesamtmenge des gereinigten Extrakts, erhalten wird.

14. Verfahren nach einem der Ansprüche 8 bis 13, dadurch gekennzeichnet, daß die Suspension der Bläschen von Liposomentyp mit der stabilisierenden Atelocollagenglycosaminoglycan-Trägerlösung zu im wesentlichen gleichen Volumsteilen vereignigt wird, wobei der Anteil an Bläschen vom Liposomentyp vorteilhafterweise zwischen 0,5 und 2 % der fertigen Basiszusammensetzung beträgt.

15. Verfahren nach einem der Ansprüche 11 bis 14, dadurch gekennzeichnet, daß man als Mukopolysaccharide Hyaluronsäure, 4-Chondroitinsulfat, 6-Chondroitinsulfat, Dermatansulfat, Keratansulfat oder Mukoitinsulfat verwendet.

16. Verfahren zur Herstellung einer kosmetischen Zusammensetzung in Form eines Haarmittels zur Behandlung der Haare, dadurch gekennzeichnet, daß sie hergestellt wird aus einer Basiszusammensetzung wie in einem der vorhergehenden Ansprüche definiert.

17. Verfahren nach Anspruch 16, dadurch gekennzeichnet, daß sie fertiggestellt wird aus der Basiszusammensetzung, indem die Suspension aus Bläschen vom Liposomentyp in einer einen Zitratpuffer bildenden wässerigen Lösung verdünnt wird, in den vorzugsweise ein Alkylenglykol, insbesondere Propylenglykol, zugegeben wird, insbesondere auf eine etwa 10-fache Verdünnung.

18. Verwendung eines Glykoproteins, insbesondere von Fibronektin oder Laminin, in Kombination mit Collagen oder Atelocollagen zur Herstellung einer Basiszusammensetzung oder einer kosmetischen Zusammensetzung zur Behandlung der Haare.
